(19) Europäisches Patentamt
European Patent Office
Office européen des brevets

(11) **EP 3 883 453 B1**

(12) **EUROPÄISCHE PATENTSCHRIFT**

(45) Veröffentlichungstag und Bekanntmachung des Hinweises auf die Patenterteilung:
**24.04.2024 Patentblatt 2024/17**

(21) Anmeldenummer: **19808750.4**

(22) Anmeldetag: **20.11.2019**

(51) Internationale Patentklassifikation (IPC):
***A61B 3/107*** *(2006.01)* ***G01B 11/25*** *(2006.01)*
*G01B 11/24* *(2006.01)*

(52) Gemeinsame Patentklassifikation (CPC):
**A61B 3/107;** G01B 11/24

(86) Internationale Anmeldenummer:
**PCT/EP2019/081926**

(87) Internationale Veröffentlichungsnummer:
**WO 2020/104525 (28.05.2020 Gazette 2020/22)**

(54) **ANORDNUNG UND VERFAHREN ZUR KOMPENSATION DER TEMPERATURABHÄNGIGKEIT EINER FACETTENLINSE FÜR DIE BESTIMMUNG DER TOPOGRAPHIE EINES AUGES**

ARRANGEMENT AND METHOD FOR COMPENSATING THE TEMPERATURE DEPENDENCE OF A FACETTED LENS FOR DETERMINING THE TOPOGRAPHY OF AN EYE

DISPOSITIF ET PROCEDE DE COMPENSATION DE LA DEPENDANCE DE LA TEMPERATURE D'UNE LENTILLE A FACETTES POUR LA DETERMINATION DE LA TOPOGRAPHIE D'UN OEIL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.11.2018 DE 102018219902**

(43) Veröffentlichungstag der Anmeldung:
**29.09.2021 Patentblatt 2021/39**

(73) Patentinhaber: **Carl Zeiss Meditec AG**
**07745 Jena (DE)**

(72) Erfinder:
• **BAJRAMOVIC, Ferid**
**94437 Mamming (DE)**
• **FUCHS, Rico**
**07745 Jena (DE)**

(74) Vertreter: **Kintzel, Klaus-Peter**
**Carl Zeiss AG**
**Patentabteilung**
**Carl-Zeiss-Promenade 10**
**07745 Jena (DE)**

(56) Entgegenhaltungen:
WO-A1-2014/074572    DE-A1-102011 102 355
US-A1- 2008 239 444    US-A1- 2012 026 466
US-A1- 2015 083 193

EP 3 883 453 B1

**Beschreibung**

Die vorliegende Erfindung betrifft eine Lösung zur Kompensation der Temperaturabhängigkeit einer Facettenlinse die zur Bestimmung der Topographie eines Auges verwendet wird.

Unter dem Begriff Keratometrie ist die Vermessung von Form und Gestalt der Hornhaut des Auges zu verstehen. Mit einem Ophthalmometer (auch Keratometer) werden die Krümmungsradien der Hornhaut zentral und in der Peripherie ermittelt. Eine besondere Form der Keratometrie ist die Topographie, bei der die zentralen und peripheren Krümmungsradien der Hornhaut mit speziellen Verfahren gemessen und mathematisch ausgewertet werden.

Verfahren zur Vermessung der Hornhautoberflächenform mit Hilfe so genannter Keratometer oder Keratographen ist nach dem Stand der Technik seit langem bekannt. Dabei wird ein auf die Hornhaut abgebildetes, vorzugsweise konzentrisches Muster durch den Tränenfilm der Hornhaut reflektiert und mit einer Kamera aufgenommen und ausgewertet. In Abhängigkeit der Kurvatur der Hornhaut ist das von der Kamera detektierte reflektierte Muster verzerrt. Um aus diesen Reflexionssignalen eine Bestimmung der Kurvatur zu erhalten, müssen die Verzerrungen des Musters mit einer bekannten Form verglichen werden, die üblicherweise als eine Kugel mit einem Radius von 7,8 mm gewählt ist.

Ein bevorzugtes System zur Bestimmung der Topographie der Kornea eines Auges wird in der DE 10 2011 102 355 A1 beschrieben. Diese Art der Topographiemessung hat den Vorteil, dass Verschiebungen des Messsystems in Bezug auf das Patientenauge sich in den Messdaten nur als Defokussierung (axiale Verschiebung) und Translation des gesamten Punktmusters im Kamerabild (laterale Verschiebung) äußern.

Da nur die relativen Abstände der Lichtpunkte Nutzinformation tragen, ist die Messung also unabhängig von einer solchen Verschiebung. Dies gilt praktisch aber nur in gewissen Grenzen, nämlich solange jedes Lichtbündel den Teil der Hornhautoberfläche trifft, für den die erforderliche Winkelbeziehung gemäß der DE 10 2014 207 058 A1 gilt. Das Raumelement, in dem dies für alle Lichtpunkte des Musters gilt, wird als "Alignmentrange" der Topographiemessung bezeichnet. Da dieses endlich groß ist, ist durch die Gerätesoftware für jede Einzelmessung zu prüfen, ob die Positionierung des Messsystems zum Patientenauge innerhalb des Alignmentrange liegt. Diese Prüfung bezeichnet man als "Alignmentcheck".

Allerdings sind die Winkel der Strahlbündel von der Temperatur der Facettenlinse abhängig. Einerseits ist es zwar vorteilhaft die Facettenlinse aus Kunststoff zu fertigen, andererseits wirken sich die Temperatureinflüsse auf Kunststoff stärker aus als auf Glas. Die temperaturabhängige Veränderung der Winkel der Strahlbündel resultiert dabei aus der Veränderung der optischen Wirkung der Facettenlinse durch Änderung der Geometrie und/oder des Brechungsindex des Kunststoffs.

Ohne eine Temperaturkompensation funktioniert die vorgeschlagene Lösung bei einer Fertigung aus Kunststoff nur in einem Temperaturbereich von etwa $(20 \pm 5)°C$. Dieser für den realen Einsatz doch recht enge Toleranzbereich macht die Lösung damit impraktikabel.

Der vorliegenden Erfindung liegt die Aufgabe zugrunde eine Lösung für die temperaturunabhängige Bestimmung der Topographie eines Auges auf der Basis einer Facettenlinse zu entwickeln. Die Facettenlinse soll dabei leicht zu fertigen sein, vorzugsweise aus Kunststoff bestehen und in einem Temperaturbereich von 10° C bis 40 °C einsetzbar sein.

Die Aufgabe zur Kompensation der Temperaturabhängigkeit einer Facettenlinse für die Bestimmung der Topographie eines Auges, bestehend aus einer Beleuchtungseinheit, einer Facettenlinse, einer Bildaufnahmeeinheit, optischen Elementen zur Trennung von Beleuchtungs- und Detektionsstrahlengang sowie einer Steuer- und Auswerteeinheit wird erfindungsgemäß dadurch gelöst, dass zusätzlich Temperatursensoren zur Bestimmung der Temperatur der Facettenlinse vorhanden sind, dass in der Steuer- und Auswerteeinheit die vorab für ein Facettenlinsendesign ermittelte Temperaturabhängigkeiten der Abstrahlwinkel der Strahlbündel hinterlegt sind und dass die Steuer- und Auswerteeinheit ausgebildet ist, bei der Auswertung der Aufnahmen der Bildaufnahmeeinheit die von den Temperatursensoren übermittelten Temperatur der Facettenlinse und die hinterlegten Temperaturabhängigkeiten der Abstrahlwinkel der Strahlbündel zu berücksichtigen.

Bevorzugte Weiterbildungen und Ausgestaltungen sind Gegenstand der abhängigen Ansprüche.

Die vorgeschlagene Lösung zur Kompensation der Temperaturabhängigkeit einer Kunststofflinse ist insbesondere für Facettenlinsen vorgesehen, die zur Bestimmung der Topographie eines Auges verwendet werden.

Die Erfindung wird nachfolgend anhand von Ausführungsbeispielen näher beschrieben. Dazu zeigen

Figur 1:    die Abhängigkeit der Strahlrichtungen von der Temperatur für 8 Ringe einer Facettenlinse,

Figur 2:    die Abhängigkeit des refraktiven Brechungsindexes n von der Temperatur T,

Figur 3:    die Abhängigkeit der Brennweite f in von der Temperatur T und

Figur 4:    Schwer- und Grenzstrahlen mehrerer Strahlbündel für drei unterschiedliche Temperaturen im Vergleich.

Die vorgeschlagene Lösung basiert auf der Verwendung einer Facettenlinse, wie sie beispielsweise in der DE 10 2011 102 355 A1 beschrieben wird. Das System besteht dabei aus der Facettenlinse, die von einer Beleuchtungseinheit mit ebenen Wellen beleuchtet wird, einer Bildaufnahmeeinheit und einer Steuer-und Auswerteeinheit. Die Bildaufnahmeeinheit ist hierbei für eine telezentrische, entfernungsunabhängige Bilderfassung ausgebildet. Wie bereits erwähnt hat diese Art der Topographiemessung den Vorteil, dass sich Verschiebungen des Messsystems in Bezug auf das Patientenauge in den Messdaten lediglich als axiale bzw. laterale Verschiebung des gesamten Punktmusters im Kamerabild äußern. Dies resultiert daraus, dass aufgrund des Messprinzips geometrisch für jeden Lichtpunkt der Normalenvektor an der Hornhautoberfläche unabhängig von der konkreten Messung bekannt ist und für die Rekonstruktion der Oberfläche dem jeweiligen Lichtpunkt durch Bestimmen des zugehörigen Referenzpunkts zugeordnet werden kann. Abhängig von Fertigungstoleranzen des Messsystems werden die Normalenvektoren als bekannt angenommen oder für jedes Einzelgerät entsprechend kalibriert.

Die vorgeschlagene Anordnung zur Kompensation der Temperaturabhängigkeit einer Facettenlinse für die Bestimmung der Topographie eines Auges besteht aus einer Beleuchtungseinheit, einer Facettenlinse, einer Bildaufnahmeeinheit, optischen Elementen zur Trennung von Beleuchtungs- und Detektionsstrahlengang sowie einer Steuer- und Auswerteeinheit.

Erfindungsgemäß sind zusätzlich Temperatursensoren zur Bestimmung der Temperatur der Facettenlinse vorhanden. Weiterhin sind in der Steuer- und Auswerteeinheit die Temperaturabhängigkeiten der Abstrahlwinkel der Strahlbündel für die Facettenlinse und damit der Normalenvektoren an der Hornhautoberfläche hinterlegt. Erfindungsgemäß ist die Steuer- und Auswerteeinheit ausgebildet, bei der Auswertung der Aufnahmen der Bildaufnahmeeinheit die von den Temperatursensoren übermittelten Temperatur der Facettenlinse und die hinterlegten Temperaturabhängigkeiten der Abstrahlwinkel der Strahlbündel zu berücksichtigen. Die Temperatursensoren sind dazu in unmittelbarer Nähe, an oder in der Facettenlinse angeordnet und werden nach der Messung ergänzend zu der Bildaufnahmeeinheit ausgelesen und gegebenenfalls abgespeichert.

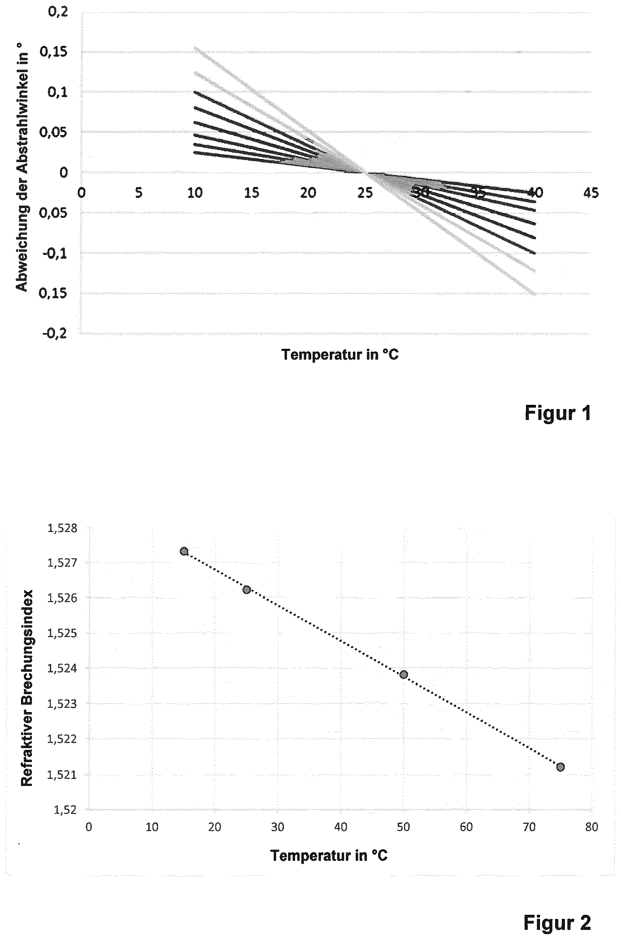

Beispielhaft ist hierzu in der **Figur 1** die Abhängigkeit der Strahlrichtungen von der Temperatur für 8 Ringe einer Facettenlinse dargestellt. Die Darstellung zeigt eine lineare Abhängigkeit der Abweichung der Strahlrichtungen. Bei einer Temperatur von 25°C treten keinerlei Abweichungen der Abstrahlwinkel der Strahlbündel der einzelnen Ringe der Facettenlinse auf.

Die in der Steuer- und Auswerteeinheit zu hinterlegenden Temperaturabhängigkeiten der Abstrahlwinkel der Strahlbündel für die Facettenlinse sind vorab für - ein Facettenlinsendesign zu ermitteln.

Solange das Design der Facettenlinse gleich bleibt ist es ausreichend ein Muster zur Determinierung der Temperaturabhängigkeit zu verwenden.

Die Bestimmung der Temperaturabhängigkeit der Abstrahlwinkel der Strahlbündel resultiert aus einer Optiksimulation und/oder aus einer Messung mittels eines Referenzkörpers.

Einer ersten Ausgestaltung entsprechend findet für die Bestimmung der Temperaturabhängigkeit der Abstrahlwinkel der Strahlbündel als Facettenlinse ein Referenzexemplar Verwendung, wobei der Referenzkörper eine präzise gefertigte Glaskugel mit bekanntem Radius ist.

Aus den Abweichungen des aufgenommenen vom erwarteten Punktmuster lassen sich die Winkel der Strahlbündel zurückrechnen. Dabei wird ein möglichst großer Temperaturbereich abgedeckt um auch erhöhter Innentemperatur des Messgerätes Rechnung zu tragen.

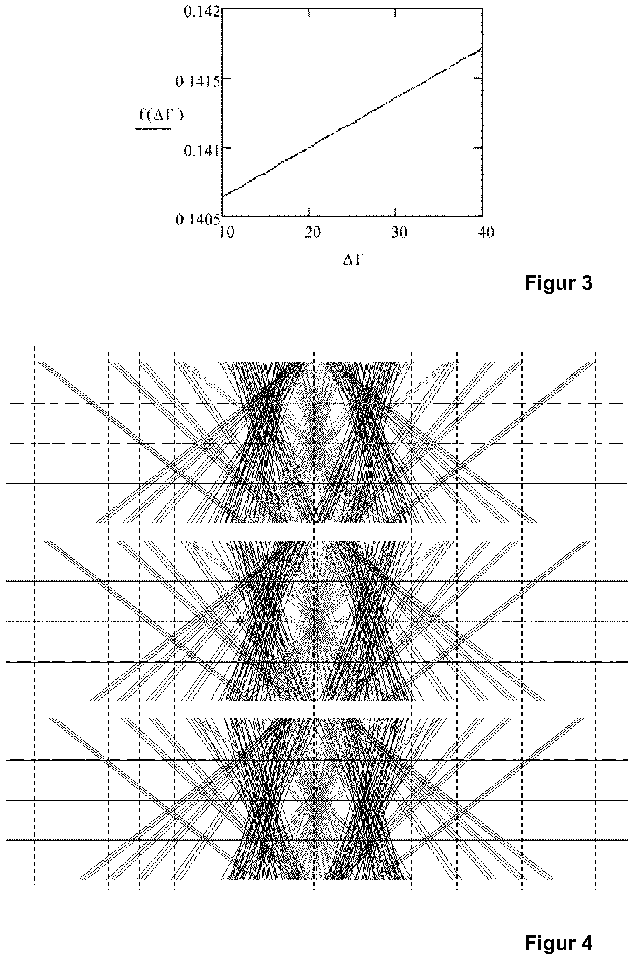

Hierzu sind in der **Figur 4** die Schwer- und Grenzstrahlen mehrerer Strahlbündel für drei unterschiedliche Temperaturen im Vergleich dargestellt. In die Abbildung wurden gestrichelte, senkrechte Hilfslinien eingefügt, um die unterschiedlichen Abstrahlwinkel der Strahlbündel der einzelnen Ringe der Facettenlinse deutlicher herauszuarbeiten. Der 3-dimensionale Schnitt der Strahlbündel beschreibt jeweils den Alignmentrange.

Bei dem vorgeschlagenen Verfahren zur Kompensation der Temperaturabhängigkeit einer Facettenlinse für die Bestimmung der Topographie eines Auges wird eine Facettenlinse von einer Beleuchtungseinheit beleuchtet, das vom Auge reflektierte Muster von einer Bildaufnahmeeinheit aufgenommen und an eine Steuer- und Auswerteeinheit weitergeleitet. Die Trennung von Beleuchtungs- und Detektionsstrahlengang erfolgt mittels optischer Elemente.

Erfindungsgemäß wird zusätzlich die Temperatur der Facettenlinse mittels Temperatursensoren bestimmt, in der Steuer- und Auswerteeinheit die Temperaturabhängigkeit der Abstrahlwinkel der Strahlbündel hinterlegt und von der Steuer- und Auswerteeinheit bei der Auswertung der Aufnahmen der Bildaufnahmeeinheit die von den Temperatursensoren übermittelten Temperatur der Facettenlinse und die hinterlegten Temperaturabhängigkeiten der Abstrahlwinkel der Strahlbündel berücksichtigt. Dazu wird die Temperatur in unmittelbarer Nähe, an oder in der Facettenlinse gemessen, wobei die Messung der Temperatur vor, während und/oder nach der Aufnahme durch die Bildaufnahmeeinheit erfolgt und an die Steuer- und Auswerteeinheit weitergeleitet und gespeichert wird.

Bezüglich der in der Steuer- und Auswerteeinheit zu hinterlegenden Temperaturabhängigkeit der Abstrahlwinkel der Strahlbündel wird auf die **Figur 1** verwiesen, die beispielhaft die Abhängigkeit der Strahlrichtungen von der Temperatur für 8 Ringe einer Facettenlinse dargestellt. Die Darstellung zeigt eine lineare Abhängigkeit der Abweichung der Strahlrichtungen. Bei einer Temperatur von 25°C treten keinerlei Abweichungen der Abstrahlwinkel der Strahlbündel der einzelnen Ringe der Facettenlinse auf.

Die Bestimmung der Temperaturabhängigkeit der Abstrahlwinkel der Strahlbündel erfolgt vorab für jede Facettenlinse durch Optiksimulation und/oder durch Messung mittels eines Referenzkörpers.

Hierbei kann die Bestimmung der Temperaturabhängigkeit durch die Beschreibung mit einer mathematischen Funktion erfolgten, wobei die mathematische Funktion vorzugsweise linear ist.

Eine Beschreibung durch eine lineare Funktion ist möglich, da der Verlauf des Brechungsindexes $\Delta n/\Delta T$ des verwendeten Materials unserer Facettenlinse im genutzten Temperaturbereich annähernd linear ist.

Hierzu zeigt die **Figur 2** die Abhängigkeit des refraktiven Brechungsindexes n von der Temperatur T, wobei diese einen linearen Verlauf aufweist.

Unter der Voraussetzung, dass die Wärmeausdehnung linear ist, ergibt sich auch für die Ausdehnung des Linsenradius R eine lineare Abhängigkeit

$$R(\Delta T) := R_0 + \alpha_{therm} \cdot R_0 \cdot \Delta T$$

in der

$R_0$     den Radius,
$\alpha_{therm}$     den linearen Ausdehnungskoeffizienten und
$\Delta T$     die Temperaturänderung der Facettenlinse definieren.

Auf der Grundlage einer Abschätzung mit einfacher dünner Linse ist damit auch die globale Wirkung, d. h. die Brennweitenwirkung linear. Aus der Änderung des Brechungsindizes $\Delta n$

$$n(\Delta T) := \Delta n \cdot \Delta T + n_{25}$$

ergibt sich die Brennweite f der Facettenlinse als Funktion der Temperatur:

$$f(\Delta T) := \frac{1}{n(\Delta T) - 1} \cdot R(\Delta T)$$

in der

R     den Radius,
n     den Brechungsindex und
$\Delta T$     die Temperaturänderung der Facettenlinse definieren.

Hierzu zeigt die **Figur 3** die Abhängigkeit der Brennweite f in von der Temperatur T, wobei diese ebenfalls einen linearen Verlauf aufweist.

Übertragen auf die Winkel der Einzelfacettenwinkel bedeutet dies, dass die Wirkung immer linear ist. Allerdings ist die Größe dieser Wirkung dabei unterschiedlich und vom jeweiligen Hauptstrahlwinkel abhängig.

Lokale Wirkungen wie die Auswirkung auf die Divergenz der eigentlich möglichst gut kollimierten Strahlbündel (ebene Wellenfront), und damit der Abstandsunabhängigkeit der Messung, lassen sich nicht temperaturkalibrieren, sind aber vermutlich wesentlich kleiner und vernachlässigbar.

Die Bestimmung der Temperaturabhängigkeit erfolgt durch die Beschreibung mit einer mathematischen Funktion, wobei die mathematische Funktion vorzugsweise logarithmisch ist. Diese alternative Option ist möglich, da in der Praxis logarithmische Temperaturabhängigkeiten existieren.

Einer ersten Ausgestaltung des Verfahrens entsprechend, werden für die Bestimmung der Temperaturabhängigkeit der Abstrahlwinkel der Strahlbündel eine Referenz-Facettenlinse und als Referenzkörper eine präzise gefertigte Glaskugel mit bekanntem Radius verwendet.

Aus den Abweichungen des aufgenommenen vom erwarteten Punktmuster lassen sich die Winkel der Strahlbündel zurückrechnen.

Um auch einer erhöhten Innentemperatur des Messgerätes Rechnung zu tragen wird die Temperaturabhängigkeit für einen zulässigen Temperaturbereich von 10°C bis 40°C bestimmt.

Hierzu sind in der **Figur 4** die Schwer- und Grenzstrahlen mehrerer Strahlbündel für drei unterschiedliche Temperaturen im Vergleich dargestellt. In die Abbildung wurden gestrichelte, senkrechte Hilfslinien eingefügt, um die unterschiedlichen Abstrahlwinkel der Strahlbündel der einzelnen Ringe der Facettenlinse deutlicher herauszuarbeiten. Der 3-dimensionale Schnitt der Strahlbündel beschreibt jeweils den Alignmentrange. Außerdem ist dadurch die Temperaturabhängigkeit besser zu sehen.

Eine zweite vorteilhafte Ausgestaltung des Verfahrens betrifft die Bestimmung der Temperaturabhängigkeit. Da die Facettenlinse weitgehend eine Rotationssymmetrie aufweist, kann die Bestimmung dahingehend vereinfacht, dass diese für einen Facettenring nur einmal erfolgt.

Allerdings kann eine Steigerung der Genauigkeit dadurch erreicht werden, dass die Bestimmung der Temperaturabhängigkeit für jeden Facettenring mehrmals erfolgt und die Messwerte gemittelt werden.

Die dritte vorteilhafte Ausgestaltung des Verfahrens betrifft eine Kalibrierung jeder Facettenlinse während der Fertigung auf eine Normaltemperatur von 25°C. Bei der ohnehin erforderlichen Kalibrierung mit einer Referenzkugel wird jede der 650 oder auch mehr einzelnen Facetten kalibriert, wobei der Einfallswinkel des respektiven Normalenvektors für jede Facette gespeichert wird. Die Funktion der Temperaturkompensation wird dann zu dieser Basistemperatur angesetzt.

Die durch Optiksimulation und/oder durch Messung mittels eines Referenzkörpers bestimmte Temperaturabhängigkeit der Abstrahlwinkel der Strahlbündel an jede Facettenlinse wird dann dieser Kalibrierung an jede einzelne Facettenlinse angepasst.

Im einfachsten Fall kann die Verrechnung linear erfolgen, wobei der konstanter Anteil aus der Kalibrierung des Einzelgeräts stammt und die Steigung der Geraden aus der bekannte Temperaturabhängigkeit laut Optikrechnung oder Referenzmessung. Daraus ergeben sich die Normalenvektoren für die temperaturkompensierte Rekonstruktion der Topographie.

Es wäre aber auch möglich weitere Funktionen zuzulassen. Dazu bieten sich beispielsweise logarithmische oder exponentielle Funktion an, da diese oft bei Temperatureffekten zu erwarten sind.

Der temperaturabhängige Alignmentrange lässt sich entweder als 3-dimensionaler Schnitt vieler Strahlbündel mit temperaturabhängigen Winkeln ermitteln (siehe **Figur 4)** oder experimentell an der Referenzkugel.

Vorzugsweise wird der Schnitt der Strahlbündel unter Verwendung der Winkelkalibrierung je Einzelgerät durchgeführt. Vereinfacht kann alternativ der Unterschied zwischen idealem Alignmentpunkt des Einzelgeräts und idealem Alignmentpunkt laut Modell bzw. Referenzgerät ermittelt und verrechnet werden.

Einer vierten vorteilhaften Ausgestaltung des Verfahrens entsprechend kann auf die Kalibrierung jeder Facettenlinse während der Fertigung verzichtet werden, wenn die Fertigungstoleranzen gering sind.

Mit der vorliegenden Erfindung wird eine Lösung für die temperaturunabhängige Bestimmung der Topographie eines Auges auf der Basis einer Facettenlinse zur Verfügung gestellt, die trotz einer leichten Fertigung aus Kunststoff in einem breiten Temperaturbereich von 10° C bis 40 °C einsetzbar ist.

Es kann sinnvoll sein, die hier beschriebenen Maßnahmen auch dann einzusetzen, wenn die Temperaturabhängigkeit durch Hardware-Maßnahmen korrigiert wird. Dies ist dadurch sinnvoll, dass mittels Software jede Facette einzeln genau kalibrieren kann, während Hardwaremaßnahmen eher auf globale Effekte adressiert sind

Je nach Ausgestaltung der Hardware-Maßnahmen verbleibenden Restfehler, die sich mit der hier beschriebenen Lösung kompensieren lassen. Die Kombination hat zudem den Vorteil, dass sich der Alignmentrange durch Temperaturänderung weniger reduziert.

Mit der vorliegenden Erfindung wird eine Lösung für die Bestimmung der Topographie eines Auges zur Verfügung gestellt, bei der die Kompensation der Temperaturabhängigkeit einer Facettenlinse auf einfache und komfortable Art und Weise realisiert.

Eine aktive Temperierung der Facettenlinse wäre zwar prinzipiell möglich aber teurer und/oder impraktikabel. Gleiches trifft auf eine Herstellung der Facettenlinse aus temperaturstabilem Material zu. Auch die Verwendung von Geräten mit einer Facettenlinse ohne Temperaturkompensation macht keinen Sinn, da diese Geräte lediglich in streng klimatisierten Räumen einsetzbar wären.

**Patentansprüche**

1. Anordnung zur Kompensation der Temperaturabhängigkeit einer Facettenlinse für die Bestimmung der Topographie eines Auges, bestehend aus einer Beleuchtungseinheit, der Facettenlinse, einer Bildaufnahmeeinheit, optischen Elementen zur Trennung von Beleuchtungs- und Detektionsstrahlengang sowie einer Steuer- und Auswerteeinheit, **dadurch gekennzeichnet, dass** zusätzlich Temperatursensoren zur Bestimmung der Temperatur der Facettenlinse vorhanden sind, dass in der Steuer- und Auswerteeinheit die vorab für ein Facettenlinsendesign ermittelte Temperaturabhängigkeiten der Abstrahlwinkel der Strahlbündel hinterlegt sind und dass die Steuer- und Auswerteeinheit ausgebildet ist, bei der Auswertung der Aufnahmen der Bildaufnahmeeinheit die von den Temperatursensoren übermittelten Temperatur der Facettenlinse und die hinterlegten Temperaturabhängigkeiten der Abstrahlwinkel der Strahlbündel zu berücksichtigen.

2. Anordnung nach Anspruch 1, **dadurch gekennzeichnet, dass** die Bestimmung der Temperaturabhängigkeit der Abstrahlwinkel der Strahlbündel aus einer Optiksimulation und/oder aus einer Messung mittels eines Referenzkörpers resultiert.

3. Anordnung nach den Ansprüchen 1 und 2, **dadurch gekennzeichnet, dass** für die Bestimmung der Temperaturabhängigkeit der Abstrahlwinkel der Strahlbündel als Facettenlinse ein Referenzexemplar Verwendung findet.

4. Anordnung nach Anspruch 3, **dadurch gekennzeichnet, dass** der Referenzkörper eine präzise gefertigte Glaskugel mit bekanntem Radius ist.

5. Verfahren zur Kompensation der Temperaturabhängigkeit einer Facettenlinse für die Bestimmung der Topographie eines Auges, bei dem eine Facettenlinse von einer Beleuchtungseinheit beleuchtet, dass vom Auge reflektierte Muster von einer Bildaufnahmeeinheit aufgenommen und an eine Steuer- und Auswerteeinheit weitergeleitet wird, wobei die Trennung von Beleuchtungs- und Detektionsstrahlengang mittels optischer Elementen erfolgt, **dadurch gekennzeichnet, dass** zusätzlich die Temperatur der Facettenlinse mittels Temperatursensoren bestimmt wird, dass in der Steuer- und Auswerteeinheit die vorab für ein Facettenlinsendesign ermittelten Temperaturabhängigkeiten der Abstrahlwinkel der Strahlbündel hinterlegt sind und dass die Steuer- und Auswerteeinheit bei der Auswertung der Aufnahmen der Bildaufnahmeeinheit die von den Temperatursensoren übermittelten Temperatur der Facettenlinse und die hinterlegten Temperaturabhängigkeiten der Abstrahlwinkel der Strahlbündel berücksichtigt.

**6.** Verfahren nach Anspruch 5, **dadurch gekennzeichnet, dass** die Messung der Temperatur vor, während und/oder nach der Aufnahme durch die Bildaufnahmeeinheit erfolgt und an die Steuer- und Auswerteeinheit weitergeleitet und gespeichert wird.

**7.** Verfahren nach Anspruch 5, **dadurch gekennzeichnet, dass** die Bestimmung der Temperaturabhängigkeit der Abstrahlwinkel der Strahlbündel durch eine Optiksimulation und/oder durch eine Messung mittels eines Referenzkörpers erfolgt.

**8.** Verfahren nach Anspruch 7, **dadurch gekennzeichnet, dass** die Bestimmung der Temperaturabhängigkeit durch Beschreibung mit einer mathematischen Funktion erfolgt.

**9.** Verfahren nach Anspruch 7, **dadurch gekennzeichnet, dass** als Referenzkörper eine präzise gefertigte Glaskugel mit bekanntem Radius verwendet wird.

**10.** Verfahren nach den Ansprüchen 5 und 7, **dadurch gekennzeichnet, dass** für die Bestimmung der Temperaturabhängigkeit der Abstrahlwinkel der Strahlbündel als Facettenlinse ein Referenzexemplar verwendet wird.

**11.** Verfahren nach den Ansprüchen 5 und 7, **dadurch gekennzeichnet, dass** die Temperaturabhängigkeit für einen zulässigen Temperaturbereich von 10°C bis 40°C bestimmt wird.

**12.** Verfahren nach den Ansprüchen 5 und 7, **dadurch gekennzeichnet, dass** die Temperaturabhängigkeit eines Facettenringes einmal bestimmt wird.

**13.** Verfahren nach den Ansprüchen 5 und 7, **dadurch gekennzeichnet, dass** die Temperaturabhängigkeit eines Facettenringes mehrmals bestimmt und die Messwerte gemittelt werden.

**14.** Verfahren nach Anspruch 5, **dadurch gekennzeichnet, dass** jede Facettenlinse während der Fertigung auf eine Normaltemperatur von 25°C kalibriert wird.

**15.** Verfahren nach den Ansprüchen 5 und 12, **dadurch gekennzeichnet, dass** die durch Optiksimulation und/oder Messung mittels eines Referenzkörpers bestimmte Temperaturabhängigkeit der Abstrahlwinkel der Strahlbündel an jede Facettenlinse, die während der Fertigung auf eine Normaltemperatur von 25°C kalibriert wurde, angepasst wird.

**Claims**

**1.** Arrangement for compensating for the temperature dependence of a facet lens for determining the topography of an eye, consisting of an illumination unit, the facet lens, an image recording unit, optical elements for separating illumination beam path and detection beam path, and a control and evaluation unit, **characterized in that** temperature sensors are additionally present for determining the temperature of the facet lens, **in that** the temperature dependences of the emission angles of the beams, ascertained in advance for a facet lens design, are stored in the control and evaluation unit, and **in that** the control and evaluation unit is designed to consider the temperature of the facet lens transferred from the temperature sensors and the stored temperature dependences of the emission angles of the beams when evaluating the recordings of the image recording unit.

**2.** Arrangement according to Claim 1, **characterized in that** the determination of the temperature dependence of the emission angles of the beams emerges from an optical simulation and/or a measurement by means of a reference body.

**3.** Arrangement according to Claims 1 and 2, **characterized in that** a reference specimen finds use as facet lens for the determination of the temperature dependence of the emission angles of the beams.

**4.** Arrangement according to Claim 3, **characterized in that** the reference body is a precisely manufactured glass sphere with a known radius.

**5.** Method for compensating for the temperature dependence of a facet lens for determining the topography of an eye, wherein a facet lens is illuminated by an illumination unit, the pattern reflected by the eye is recorded by an image recording unit and transmitted to a control and evaluation unit, the illumination beam path and detection beam path being separated by means of optical elements, **characterized in that** the temperature of the facet lens is additionally determined by means of temperature sensors, **in that** the temperature dependences of the emission angles of the beams, ascertained in advance for a facet lens design, are stored in the control and evaluation unit, and **in that** the control and evaluation unit considers the temperature of the facet lens transferred from the temperature sensors and the stored temperature dependences of the emission angles of the beams when evaluating the recordings of the image recording unit.

**6.** Method according to Claim 5, **characterized in that** the temperature is measured before, during and/or after the recording carried out by the image recording unit and transmitted to and stored by the control and evaluation unit.

7. Method according to Claim 5, **characterized in that** the determination of the temperature dependence of the emission angles of the beams is implemented by way of an optical simulation and/or by way of a measurement by means of a reference body.

8. Method according to Claim 7, **characterized in that** the temperature dependence is determined by way of a description using a mathematical function.

9. Method according to Claim 7, **characterized in that** a precisely manufactured glass sphere with a known radius is used as a reference body.

10. Method according to Claims 5 and 7, **characterized in that** a reference specimen is used as facet lens for the determination of the temperature dependence of the emission angles of the beams.

11. Method according to Claims 5 and 7, **characterized in that** the temperature dependence is determined for an admissible temperature range from 10°C to 40°C.

12. Method according to Claims 5 and 7, **characterized in that** the temperature dependence of a facet ring is determined once.

13. Method according to Claims 5 and 7, **characterized in that** the temperature dependence of a facet ring is determined multiple times and the measurement values are averaged.

14. Method according to Claim 5, **characterized in that** each facet lens is calibrated during manufacture to a normal temperature of 25°C.

15. Method according to Claims 5 and 12, **characterized in that** the temperature dependence of the emission angles of the beams determined by an optical simulation and/or a measurement by means of a reference body is adapted to each facet lens which was calibrated during manufacture to a normal temperature of 25°C.

**Revendications**

1. Ensemble destiné à compenser la dépendance à la température d'une lentille à facettes destinée à déterminer la topographie d'un œil, ledit ensemble comprenant une unité d'éclairage, la lentille à facettes, une unité d'acquisition d'images, des éléments optiques destinés à séparer les trajets de rayons d'éclairage et de détection et une unité de commande et d'évaluation, **caractérisé en ce que** des capteurs de température sont en plus prévus pour déterminer la température de la lentille à facettes, **en ce que** les dépendances à la température des angles de rayonnement des faisceaux de rayons, lesquelles dépendances sont déterminées à l'avance pour la conception des lentilles à facettes, sont mémorisées dans l'unité de commande et d'évaluation et **en ce que** l'unité de commande et d'évaluation est conçue pour prendre en compte la température de la lentille à facettes transmise par les capteurs de température et les dépendances à la température mémorisée des angles de rayonnement des faisceaux de rayons, lors de l'évaluation des acquisitions faites par l'unité d'acquisition d'images.

2. Ensemble selon la revendication 1, **caractérisé en ce que** la détermination de la dépendance à la température de l'angle de rayonnement des faisceaux de rayons résulte d'une simulation optique et/ou d'une mesure à l'aide d'un corps de référence.

3. Ensemble selon les revendications 1 et 2, **caractérisé en ce qu'**un échantillon de référence est utilisé comme lentille à facettes pour déterminer la dépendance à la température des angles de rayonnement des faisceaux de rayons.

4. Ensemble selon la revendication 3, **caractérisé en ce que** le corps de référence est une boule de verre de rayon connu fabriquée avec précision.

5. Procédé de compensation de la dépendance à la température d'une lentille à facettes destinée à déterminer la topographie d'un œil, procédé dans lequel une lentille à facettes est éclairée par une unité d'éclairage, les motifs réfléchis par l'œil sont acquis par une unité d'acquisition d'images et transmis à une unité de commande et d'évaluation, la séparation du trajet de rayons d'éclairage et de détection étant effectuée au moyen d'éléments optiques, **caractérisé en ce que** la température de la lentille à facettes est en outre déterminée au moyen de capteurs de température, **en ce que** les dépendances à la température des angles de rayonnement des faisceaux de rayons, lesquelles dépendances sont déterminées à l'avance pour la conception de lentille à facettes, sont mémorisées dans l'unité de commande et d'évaluation et **en ce que** l'unité de commande et d'évaluation prend en compte la température de la lentille à facettes transmise par les capteurs de température et les dépendances à la température mémorisés des angles de rayonnement des faisceaux de rayons lors de l'évaluation des acquisitions faites par l'unité d'acquisition d'images.

6. Procédé selon la revendication 5, **caractérisé en ce**

**que** la mesure de la température est effectuée avant, pendant et/ou après l'acquisition par l'unité d'acquisition d'images et est transmise à l'unité de commande et d'évaluation et mise en mémoire.

7. Procédé selon la revendication 5, **caractérisé en ce que** la dépendance à la température de l'angle de rayonnement des faisceaux de rayons est déterminée par une simulation optique et/ou par une mesure à l'aide d'un corps de référence.

8. Procédé selon la revendication 7, **caractérisé en ce que** la dépendance à la température est déterminée par une description faite avec une fonction mathématique.

9. Procédé selon la revendication 7, **caractérisé en ce qu'**une bille de verre de rayon connu fabriquée avec précision est utilisée comme corps de référence.

10. Procédé selon les revendications 5 et 7, **caractérisé en ce qu'**un exemple de référence est utilisé comme lentille à facettes pour déterminer la dépendance à la température des angles de rayonnement des faisceaux de rayons.

11. Procédé selon les revendications 5 et 7, **caractérisé en ce que** la dépendance à la température est déterminée pour une gamme de températures admissible de 10 °C à 40 °C.

12. Procédé selon les revendications 5 et 7, **caractérisé en ce que** la dépendance à la température d'un anneau à facettes est déterminée une seule fois.

13. Procédé selon les revendications 5 et 7, **caractérisé en ce que** la dépendance à la température d'un anneau à facettes est déterminée plusieurs fois et les valeurs de mesure sont moyennées.

14. Procédé selon la revendication 5, **caractérisé en ce que** chaque lentille à facettes est calibrée à une température normale de 25 °C lors de la fabrication.

15. Procédé selon les revendications 5 et 12, **caractérisé en ce que** la dépendance à la température de l'angle de rayonnement des faisceaux de rayons, laquelle dépendance est déterminée par simulation optique et/ou mesure à l'aide d'un corps de référence, est adaptée à chaque lentille à facettes qui a été calibrée pour une température normale de 25 °C lors de la fabrication.

**Figur 1**

**Figur 2**

Figur 3

Figur 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102011102355 A1
- DE 102014207058 A1